Europäisches Patentamt

European Patent Office

Office européen des brevets

(19)

(11) EP 0 624 373 B1

(12) EUROPÄISCHE PATENTSCHRIFT

(45) Veröffentlichungstag und Bekanntmachung des Hinweises auf die Patenterteilung:
**08.09.1999 Patentblatt 1999/36**

(51) Int. Cl.⁶: $A61K\ 33/24$, $A61K\ 31/43$
// ($A61K31/43$, $31:29$)

(21) Anmeldenummer: 93810342.1

(22) Anmeldetag: **11.05.1993**

(54) **Festes bismut- und amoxycillinhaltiges Arzneipräparat und seine Verwendung**

Pharmaceutical preparation containing bismuth and amoxycillin and its use

Composition pharmaceutique contenant du bismuth et de l'amoxycillin et son utilisation

(84) Benannte Vertragsstaaten:
**AT BE CH DE DK ES FR GB GR IE IT LI LU MC NL PT SE**

(43) Veröffentlichungstag der Anmeldung:
**17.11.1994 Patentblatt 1994/46**

(73) Patentinhaber:
**SPIRIG AG
PHARMAZEUTISCHE PRÄPARATE
CH-4622 Egerkingen (CH)**

(72) Erfinder:
- **Birrenbach, Gerd
  CH-4616 Kappel (SO) (CH)**
- **Juch, Rolf-Dieter
  CH-4612 Wangen b. Olten (CH)**

(74) Vertreter: **Braun, André, jr.
BRAUN & PARTNER
Patent-, Marken-, Rechtsanwälte
Reussstrasse 22
4054 Basel (CH)**

(56) Entgegenhaltungen:
**WO-A-86/05981          WO-A-92/11848**

**Beschreibung**

Die Erfindung betrifft ein festes Arzneipräparat, insbesondere in Tablettenform, das als Wirkstoffe eine Kombination aus Bismut, vorzugsweise Bismut(III)-citrat, und Amoxycillin, vorzugsweise Amoxycillin-Trihydrat enthält.

Bismutverbindungen werden schon seit langem zur Behandlung bei Magenbeschwerden eingesetzt. Insbesondere seit der Wiederentdeckung des Helicobacter pylori als Mitverursacher von Gastritis und Magengeschwüren werden die anerkanntermassen nützlichen Bismutsalze wieder vermehrt zur Behandlung eingesetzt. Im wesentllichen handelt es sich dabei um das Bismut-Subsalicylat, -subcitrat oder -citrat, -aluminat, -nitrat oder -gallat. Bei oraler Verabreichung können die Bismut-Anteile der Präparate je nach pH im Magen kolloidale Lösungen, stark hydratisierte Gele oder dünne Filme im Magen bilden. Durch Resorption schon von geringen Mengen von Bismut im Darm und Speicherung im Körpergewebe kann es aber zu Intoxikationserscheinungen (z.B. "Bismut-Enzephalopathie" ) kommen. Es wird deshalb empfohlen, Behandlungen mit Bismutsalzen abzubrechen, wenn eine Bismutkonzentration im Blut von 50-100 ng/ml überschritten wird.

Flüssige Präparate, die Bismut enthalten, sind bekannt, vgl. EP 217 440 oder EP 363 502, und ebenso bismuthaltige Filmtabletten, vgl. EP 75 992. Die dort offenbarte Formulierung weist als Nachteil einen hohen Bismut-Peak etwa 30 Minuten nach Einnahme des Präparats im Plasma auf. Dieser Nachteil kann gemäss EP 437 294 durch eine geringere Pakkungsdichte des Bismutsalzes (kolloidales festes Bismutsubcitrat) in der pharmazeutischen Formulierung verhindert werden. Damit werden die Tabletten jedoch so voluminös, dass sie nur noch als Kautabletten angeboten werden können, was wiederum zur Schwarzfärbung der Zähne (Bildung von Bismutsulfid) und wegen des unangenehmen Geruchs und Geschmacks von Amoxycillin zu mangelnder Einnahmebereitschaft beim Patienten führen kann.

Obwohl Bismutsalze zur Behandlung der durch Helicobacter pylori verursachten Gastritis wirksam sind, bereitet das Wiederkehren der Krankheit, das sogenannte Rezidiv, grosse Probleme. Man hat deshalb versucht, das Problem durch Zugabe eines Antibiotikums zu lösen. In Vergleichsstudien führte eine Monotherapie mit Bismutpräparaten in etwa 20% zur Eradikation von Helicobacter pylori, während eine Kombinationsbehandlung mit Amoxycillin in etwa 50% zur Eradikation führte (Rauws E.A.J., G.N.J. Tytgat: Eradication of Helicobacter pylori cures duodenal ulcer. Lancet 1990/I, 1233). Bei solchen Doppeltherapien werden die Bismutsalze und Amoxycillin oder auch Tetracycline getrennt verabreicht, wobei z.B. die Amoxycillin-Dosis drei- oder viermal 500 mg täglich beträgt. Diese Doppeltherapien verlangen vom Patienten jedoch ein genaues Befolgen der Verabreichungsvorschriften, was nicht in jedem Fall gewährleistet werden kann und was wegen der Anzahl und dem Volumen der verschiedenen Tabletten auf alle Fälle aufwendig und unangenehm ist. Die Patienten-Compliance leidet in beträchtlichem Ausmass unter diesen Verhältnissen.

Die Kombination der beiden Wirkstoffe in einem als ganzes schluckbaren Arzneipräparat war bisher nicht konkretisierbar, weil einerseits das Amoxycillin mit dem Bismutsalz unter Schwarzfärbung des Gemisches reagiert und somit nicht stabil ist und andererseits das Bismutsalz gemäss dem Stand der Technik, wie er in der EP 437 294 beschrieben wird, in einer niedrigen Packungsdichte im Arzneipräparat enthalten sein muss. Es war somit anzunehmen, dass eine Kombination beider Wirkstoffe nur mit der Beimengung von grossen Mengen an Hilfsstoffen möglich ist, denn sowohl niedrige Pakkungsdichte des Bismutsalzes als auch die mangelnde chemische Stabilität der Kombination beider Wirkstoffe liess nur diesen Schluss zu. Damit würde das Volumen des Arzneipräparats keine als ganzes schluckbare Formulierung mehr zulassen, was aber zur Vereinfachung der Therapie unerlässlich ist.

Aufgabe der Erfindung ist es deshalb, ein festes Kombinationspräparat aus einem Bismutsalz und Amoxycillin zu entwickeln, das stabil und trotzdem kleinvolumig ist, so dass es ganz, d.h. unzerkaut geschluckt werden kann, wobei Bismut nur in möglichst geringem Umfang resorbiert werden soll.

Gelöst wird diese Aufgabe durch ein festes Arzneipräparat, vorzugsweise in Tablettenform, das pulverförmiges Bismut(III)-subcitrat und Amoxycillin-Trihydrat und geeignete Hilfsstoffe, wie z.B. Füllmittel, Bindemittel, Sprengmittel, Fliess- und/oder Gleitmittel enthält.

Die vorliegende Erfindung ist in den Patentansprüchen definiert. Insbesondere betrifft die vorliegende Erfindung ein festes Arzneipräparat in Tablettenform, enthaltend als Wirkstoffe eine Kombination von Bismut(III)-citrat und Amoxycillin, welches dadurch gekennzeichnet ist, dass (i) als Bismut(III)-citrat ein Bismut(III)-subcitrat und als Amoxycillin das Amoxycillin-Trihydrat verwendet werden, wobei das Bismut(III)-subcitrat in einer Konzentration von 32-36 Gew.-% und das Amoxycillin-Trihydrat in einer Konzentration von 39-43 Gew.-%, in der Tablette anwesend ist, und der verbleibende Tablettenanteil aus einem Bindemittel, Füllmittel, Sprengmittel und/oder Fliess- resp. Gleitmittel besteht.

Als Füllmittel eignet sich insbesondere mikrokristaliine Cellulose oder Laktose oder ein Gemisch davon, als Bindemittel Polyvinylpyrrolidon, Cellulosederivate, wie Hydroxypropylmethylcellulose, Gelatine, oder Polyvinylpyrrolidon-Vinylacetat Copolymere oder ein Gemisch davon, als Sprengmittel Natriumcarboxymethylcellulose, Stärke oder Stärkederivate, quervernetztes Methacrylsäuredivinylbenzol oder quervernetztes Polyvinylpyrrolidon oder ein Gemisch davon und/oder als Gleitmittel resp. Fliessmittel kolloidale Kieselsäure, Metallseifen, wie Magnesiumstearat, hydrierte Triglyceride, Cutina HR (=gehärtetes Ricinusöl) oder Talk oder ein Gemisch davon.

Als Hilfsstoffe eignen sich vorzugsweise Polyvinylpyrrolidon, mikrokristalline Cellulose, Natriumcarboxymethylcellulose, Magnesiumstearat und kolloide Kieselsäure.

Das Arzneipräparat enthält vorzugsweise 32-36 Gew.-% Bismut(III)-citrat, 39-43 Gew.-% Amoxicyllin-Trihydrat, 0,1-10 Gew.-%, besonders bevorzugt 0,5-2 Gew.-% Bindemittel, 5-25 Gew.-%, besonders bevorzugt 12-16 Gew.-% Füllmittel, 1-15 Gew.-%, besonders bevorzugt 5-9 Gew.-% Sprengmittel, 0,1-6 Gew.%, besonders bevorzugt 1-3 Gew.-% Fliess- resp. Gleitmittel.

Eine besonders bevorzugte Ausführungsform des Arzneipräparats enthält 32-36 Gew.-%, vorzugsweise 34 Gew.-% Bismut(III)-subcitrat, 39-43 Gew.-%, vorzugsweise 41 Gew.-% Amoxicyllin-Trihydrat, 0,5-2 Gew.-%, vorzugsweise 1 Gew.-% Polyvinylpyrrolidon, 12-16 Gew.-%, vorzugsweise 14 Gew.-% mikrokristalline Cellulose, 5-9 Gew.-%, vorzugsweise 7 Gew.-% Natriumcarboxymethylcellulose, und als Fliess- resp. Gleitmittel 1-3 Gew.% eines Gemisches aus vorzugsweise 3 Gew.-Teilen Magnesiumstearat und vorzugsweise 2 Gew.-Teilen kolloidale Kieselsäure.

Das vorzugsweise in Tablettenform zur Verfügung gestellte Arzneipräparat sollte ein Totalgewicht von vorzugsweise weniger als 900 mg aufweisen und enthält 80-160 mg, vorzugsweise 120 mg, Bi als $Bi_2O_3$ gemessen, und 150-350 mg, vorzugsweise 250 mg, Amoxicyllin als Base gemessen. Eine bevorzugte Tablette weist ein Gewicht von 700-900 mg und eine Härte von 12-17 kp, vorzugsweise 15 kp auf und zerfällt in Wasser von 37°C in weniger als 3 Minuten, vorzugsweise in etwa 1 Minute gemäss USP XXII. Üblicherweise werden 3-4 mal täglich zwei solcher Arzneipräparate zur Behandlung von Helicobacter pylori bedingten gastro-duodenalen Erkrankungen, wie Gastritis, Magenulcus, Duodenalulcus oder nicht-ulzeröser Dyspepsie verabreicht.

Das Arzneipräparat wird vorzugsweise als Tablette hergestellt, indem man die beiden aktiven Wirkstoffe und die Hilfsmittel auf herkömmliche Art miteinander mischt und das Gemisch auf einer Rundläufertablettenpresse direkt zu Tabletten verpresst.

Diese Tabletten zeichnen sich durch grosse Kompaktheit aus, d.h. sie enthalten wenig Hilfsstoffe, sind sehr hart und weisen ausgezeichnete Friabilitätswerte auf. Trotzdem zerfallen sie rasch und weisen eine Langzeitstabilität von drei Jahren und mehr auf. Zudem wird das im erfindungsgemässen Arzneipräparat vorhandene Bismut durch den Körper kaum resorbiert.

Die Erfindung wird im folgenden beispielsweise näher erläutert.

Beispiel 1

Die folgenden pulverförmigen Komponenten werden miteinander gemischt:

| Bismutcitrat | 244 g |
|---|---|
| Amoxycillin Trihydrat | 295 g |
| Polyvinylpyrrolidon | 8 g |
| Mikrokristalline Cellulose | 100 g |
| NaCMC | 50 g |
| Magnesiumstearat | 11 g |
| Kolloidale Kieselsäure | 6,75 g |
| | 714,75 g |

Das Gemisch wird auf einer Rundläuferpresse zu runden, bikonvexen Tabletten mit einem Durchmesser von 12 mm verpresst.

Beispiel 2

Die folgenden pulverförmigen Komponenten werden miteinander gemischt:

| Bismutcitrat | 500 g |
|---|---|
| Amoxycillin Trihydrat | 295 g |
| Polyvinylpyrrolidon | 8 g |
| Mikrokristalline Cellulose | 50 g |
| Maisstärke | 50 g |
| Magnesiumstearat | 5 g |
| Kolloidale Kieselsäure | 6,5 g |
| | 914,5 g |

Das Gemisch wird auf einer Rundläuferpresse zu runden, biplanen Tabletten mit einer Teilkerbe und einem Durchmesser von 14 mm verpresst.

Beispiel 3

Die folgenden pulverförmigen Komponenten werden miteinander gemischt:

| Bismutcitrat | 120 g |
|---|---|
| Amoxycillin Trihydrat | 600 g |

(fortgesetzt)

| Hydroxypropylmethylcellulose | 10 g |
|---|---|
| Lactose | 60 g |
| vernetztes Polyvinylpyrrolidon | 40 g |
| Cutina HR | 6 g |
| Kolloidale Kieselsäure | 7 g |
| | 843 g |

Das Gemisch wird auf einer Rundläuferpresse zu runden, bikonvexen Tabletten mit einem Durchmesser von 12 mm verpresst.

Beispiel 4

Die folgenden pulverförmigen Komponenten werden miteinander gemischt:

| Bismutcitrat | 244 g |
|---|---|
| Amoxycillin Trihydrat | 295 g |
| Gelatine | 10 g |
| Lactose | 75 g |
| Mikrokristalline Cellulose | 75 g |
| Maisstärke | 60 g |
| Magnesiumstearat | 6 g |
| Kolloidale Kieselsäure | 7 g |
| | 272 g |

Das Gemisch wird auf einer Rundläuferpresse zu runden, bikonvexen Tabletten mit einem Durchmesser von 12 mm verpresst.

Beispiel 5

An 8 gesunden Probanden wurde die Bioverfügbarkeit von Bismut und Amoxicillin nach kombinierter Verabreichung der beiden Wirkstoffe mittels Tabletten gemäss Beispiel 1 untersucht. Durch Einnahme von jeweils 2 Tabletten betrug die Gesamtdosis an Bismut 240 mg ($Bi_2O_3$) und an Amoxycillin 500 mg (Amoxycillin Base).

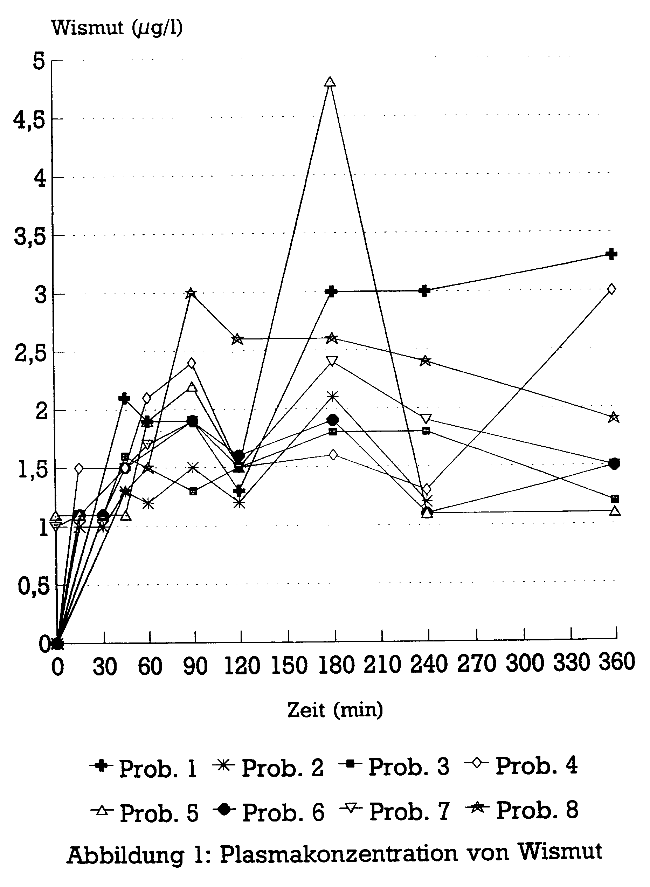

Durch Blutentnahme unmittelbar vor (=0-Wert) sowie 15, 30, 45, 60, 90, 120, 180, 240 und 360 Minuten nach der Einnahme der Tabletten wurden die Plasmawerte von Bismut quantitativ bestimmt (Abbildung 1).

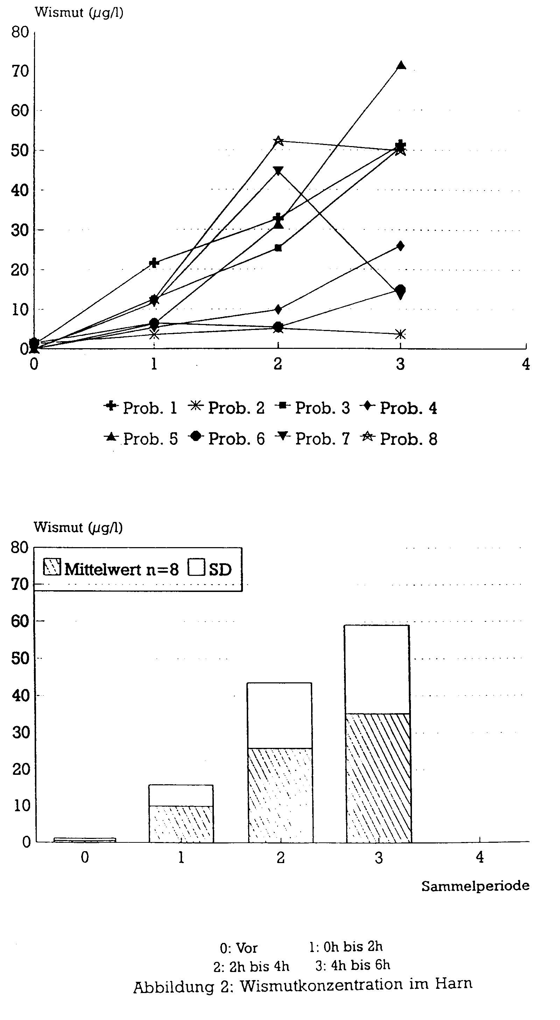

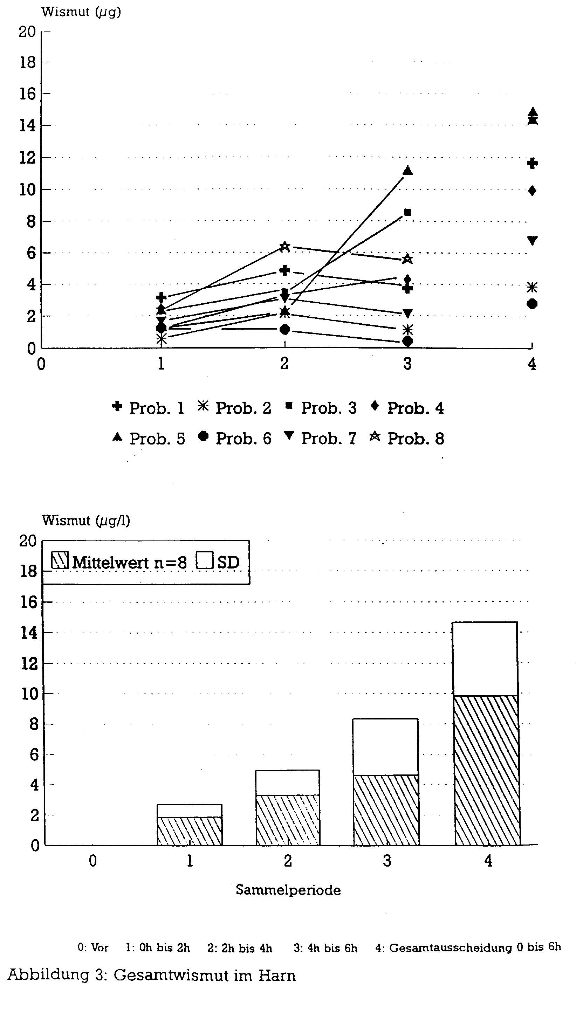

Durch Harnentnahme vor Einnahme des Präparats (=0-Wert) und 2, 4 und 6 Stunden nach Applikation wurde die Bismutkonzentration im Harn gemessen (Abbildung 2) und die Gesamtausscheidung von Bismut im Harn bis 6 Stunden nach Verabreichung (Abbildung 3).

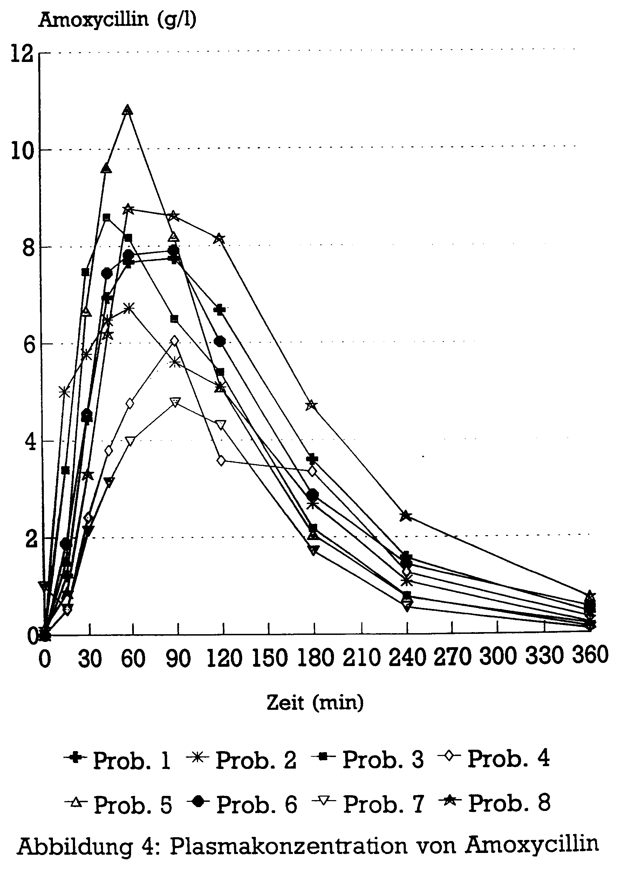

Durch Blutentnahme unmittelbar vor (=0-Wert) sowie 15, 30, 45, 60, 90, 120, 180, 240 und 360 Minuten nach der Einnahme der Tabletten wurden die Plasmawerte von Amoxicyllin quantitativ bestimmt (Abbildung 4).

Die gemessenen Plasmakonzentrationen von Amoxycillin befinden sich im Bereich, der nach Verabreichung von 500 mg Wirkstoff zu erwarten war. Dies weist darauf hin, dass die Bioverfügbarkeit von Amoxycillin aus der Bismut-Amoxycillin-Kombinationstablette sich nicht von der Bioverfügbarkeit einer nur Amoxycillin enthaltenden Tablette unterscheidet.

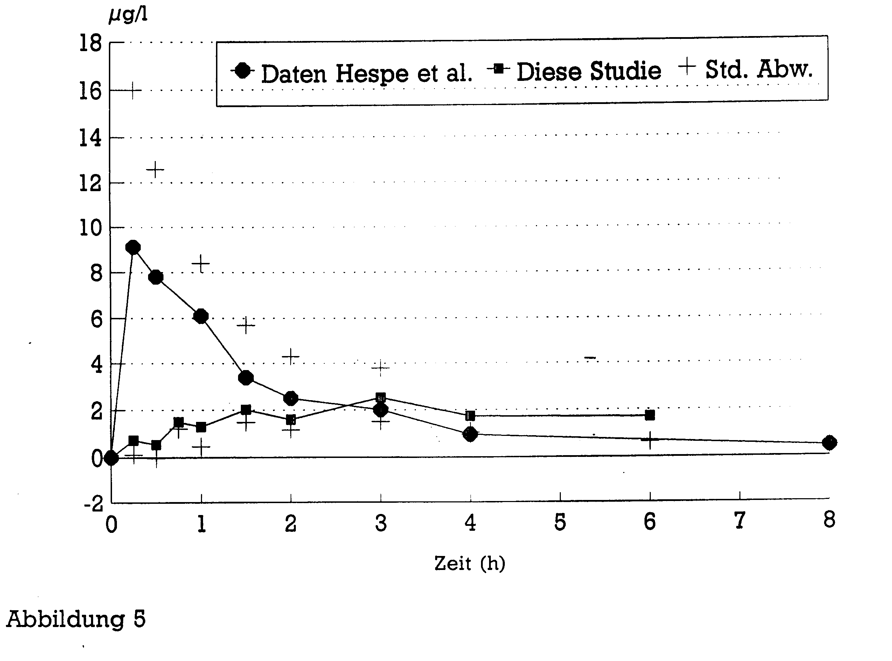

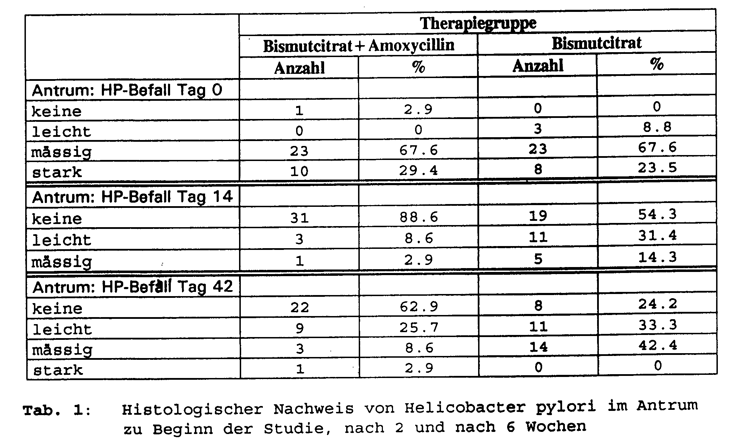

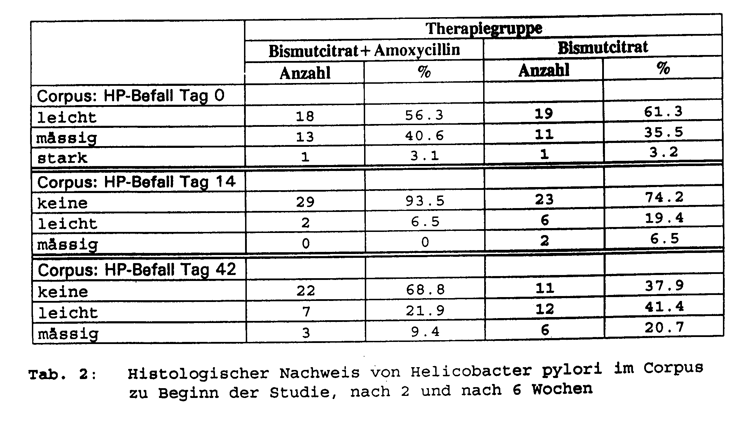

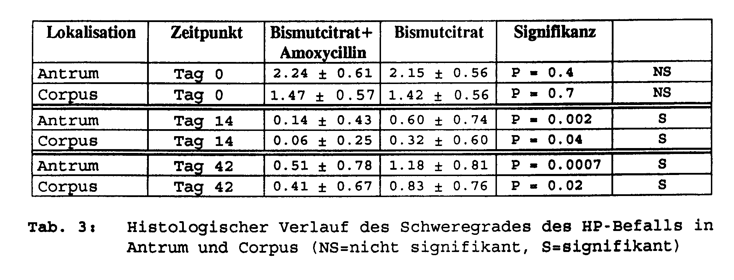

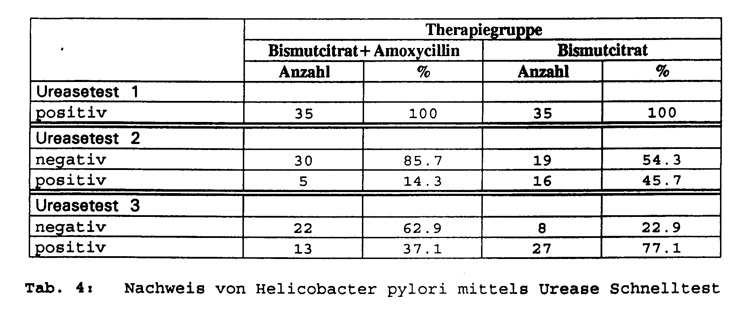

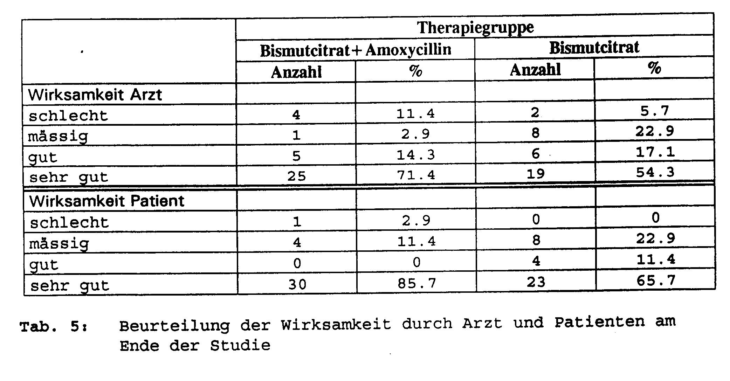

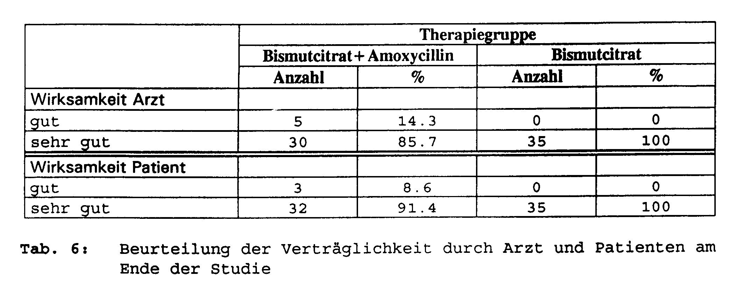

Der mittels Atomabsorptionsspektroskopie gemessene zeitliche Verlauf der Plasmakonzentration von Bismut zeigt im Vergleich mit dem von Hespe W, Staal HJM, Hall DWR (1988), "Bismuth absorption from the colloidal subcitrate", Lancet 26.11.88: 1258, gemessenen Verlauf signifikant geringere Werte auf (Abbildung 5).

Beispiel 6

Es wurde eine randomisierte Doppelblindstudie zur Eradikation von Helicobacter pylori bei nicht ulzeröser Dyspepsie an insgesamt 70 Patienten durchgeführt, bei denen eine Infektion mit Helicobacter pylori nachweislich vorlag. Als Arzneipräparat wurden der Gruppe 1 (35 Patienten) schnell zerfallende Tabletten gemäss Beispiel 1 mit 295 mg Amoxycillin-Trihydrat (entspricht 250 mg Amoxycillin-Base) und 244 mg Bismutcitrat (entspricht 120 mg Bismut als $Bi_2O_3$) 3x2 Tabletten täglich während 14 Tagen und der Gruppe 2 (35 Patienten) schnell zerfallende Tabletten mit 244 mg Bismutcitrat (entspricht 120 mg Bismut als $Bi_2O_3$) 3x2 Tabletten täglich während 14 Tagen verabreicht.

Zu Beginn der Studie, nach 2 und 6 Wochen wurde die Magenmucosa makroskopisch mittels einer 4-stufigen Skala bezüglich Erythemen und Erosionen beurteilt und die Floridität der Gastritis untersucht. Es wurde je eine Biopsie im Antrum und im Corpus entnommen und auf Heliobacterbefall histologisch untersucht. An einer weiteren Gewebeprobe wurde ein Ureasetest zum Schnell-Nachweis von Heliobacter pylori durchgeführt.

Als Kriterien für die Wirksamkeit wurden insbesondere der histologische Biopsiebefund und das Ergebnis im Ureasetest herangezogen.

In Tabelle 1 wird der histologische Nachweis von Helicobacter pylori im Antrum zu Beginn der Studie, nach 2 und nach 6 Wochen gezeigt.

In Tabelle 2 wird der histologische Nachweis von Helicobacter pylori im Corpus zu Beginn der Studie, nach 2 und nach 6 Wochen gezeigt.

In Tabelle 3 wird der histologische Verlauf des Schweregrades des Helicobacter pylori-Befalls im Antrum und Corpus (NS=nicht signifikant; S=signifikant) zu Beginn der Studie, nach 2 und nach 6 Wochen gezeigt.

Vor Studienbeginn konnte bei allen 70 Patienten Helicobacter pylori histologisch nachgewiesen werden. Nach 14 Tagen waren 31 Patienten (89%) in Gruppe 1 und 19 Patienten (54%) in Gruppe 2 im Magenantrum von Helicobacter pylori befreit. Im Corpus waren es 29 (94%) in Gruppe 1 und 23 (74%) in Gruppe 2. Weitere 4 Wochen später war Helicobacter pylori bei 22 Patienten (63%) in Gruppe 1 und bei nur 8 Patienten (24%) in Gruppe 2 im Antrum und bei 22 (69%) in Gruppe 1 und 11 (38%) in Gruppe 2 im Corpus nicht nachweisbar. Die Eliminationsrate war somit in Gruppe 1 signifikant höher als in Gruppe 2.

In Tabelle 4 wird der Nachweis von Helicobacter pylori mittels Urease Schnelltest gezeigt.

Von den 70 Patienten zeigten in Gruppe 1 nach 14 Tagen noch 5 einen positiven Urease-Test, in Gruppe 2 immerhin noch 16. Nach 6 Wochen zeigten in Gruppe 1 13 Patienten einen positiven Urease-Test, in Gruppe 2 dagegen 27.

Ferner wurde die Floridität der Gastritis bei den Patienten histologisch untersucht. Die Patienten der Gruppe 1 hatten zu Beginn signifikant aktivere Gastritiden im Antrum. Nach 14 Tagen waren die Biopsiebefunde etwa ausgeglichen, nach 6 Wochen zeigten die Biopsien der Gruppe 1 signifikant geringere Entzündungsaktivität. Im Corpus konnten hingegen keine signifikanten Unterschiede zwischen den beiden Gruppen nachgewiesen werden.

Die makroskopische Beurteilung der Magenschleimhaut zu Beginn, nach 2 und nach 6 Wochen der Studie zeigte, dass sich diese im Verlauf der Behandlung normalisierte. Bei beiden Gruppen nahmen die Schleimhauterosionen ab. Ein signifikanter Unterschied zwischen den Behandlungsgruppen liess sich nicht feststellen.

Die Beurteilung der Wirksamkeit durch Arzt und Patienten am Ende der Studie ist in Tabelle 5 festgehalten. Nach 4-wöchiger Nachbeobachtungszeit wurde die Wirksamkeit in Gruppe 1 vom Arzt und den Patienten jeweils 30 mal (86%) als "gut" bis "sehr gut" beurteilt, wohingegen in Gruppe 2 der Arzt 25 mal (71%) mit "gut" bis "sehr gut" urteilte, die Patienten 27 mal (77%).

Die Gesamtbeurteilung der Verträglichkeit ist in Tabelle 6 festgehalten. Am Ende der Studie bewerteten in Gruppe 1 die Ärzte die Verträglichkeit 5 mal als "gut" und 25 mal als "sehr gut". In Gruppe 2 urteilten sie 35 mal mit "sehr gut". 3 Patienten der Gruppe 1 fanden das Präparat "gut" verträglich, 32 "sehr gut". In Gruppe 2 waren es 35 mit "sehr gut".

Die beiden Hauptkriterien "histologischer Helicobacter pylori-Nachweis" und "Urease-Test" ergaben eine klare Überlegenheit des erfindungsgemässen Kombinationspräparats im Verhältnis zur Behandlung mit Bismutcitrat allein. Die gleiche Feststellung lässt sich zum Entzündungsgrad der Gastritiden und der Beurteilung der Wirksamkeit durch den Arzt und die Patienten machen, während die Verträglichkeit durch die Nebenwirkungen von Amoxycillin im Kombinationspräparat wie vermutet leicht geringer ausfiel.

## Patentansprüche

1. Festes Arzneipräparat in Tablettenform, enthaltend als Wirkstoffe eine Kombination von Bismut(III)-citrat und Amoxycillin, dadurch gekennzeichnet, dass (i) als Bismut(III)-citrat ein Bismut(III)-subcitrat und als Amoxycillin das Amoxycillin-Trihydrat verwendet werden, wobei das Bismut(III)-subcitrat in einer Konzentration von 32-36 Gew.-% und das Amoxycillin-Trihydrat in einer Konzentration von 39-43 Gew.-%, in der Tablette anwesend ist, und der verbleibende Tablettenanteil aus einem Bindemittel, Füllmittel, Sprengmittel und/oder Fliess- resp. Gleitmittel besteht.

2. Arzneipräparat nach Patentanspruch 1, dadurch gekennzeichnet, dass dieses als Füllmittel mikrokristalline Cellulose oder Laktose oder ein Gemisch derselben, als Bindemittel Polyvinylpyrrolidon, Cellulosederivate, vorzugsweise Hydroxypropylmethylcellulose, Gelatine oder Polyvinylpyrrolidon-Vinylacetat-Copolymere oder ein Gemisch derselben, als Sprengmittel Natriumcarboxymethylcellulose, Stärke oder Stärkederivate, quervernetztes Methacrylsäuredivinylbenzol oder quervernetztes Polyvinylpyrrolidon oder ein Gemisch derselben und als Gleitmittel resp. Fliessmittel kolloidale Kieselsäure, Metallseifen, wie Magnesiumstearat, hydrierte Triglyceride oder Talk oder ein Gemisch derselben enthält.

3. Arzneipräparat nach einem der Patentansprüche 1 oder 2, dadurch gekennzeichnet, dass dieses als Hilfsstoffe ein Gemisch aus Polyvinylpyrrolidon, mikrokristalliner Cellulose, Natriumcarboxymethylcellulose, Magnesiumstearat und kolloidaler Kieselsäure enthält

4. Arzneipräparat nach einem der Patentansprüche 1-3, dadurch gekennzeichnet, dass dieses 0,1-10 Gew.-% Bindemittel, 5-25 Gew.-% Füllmittel, 1-15 Gew.-% Sprengmittel, 0,1-6 Gew.% Fliess- resp. Gleitmittel enthält.

5. Arzneipräparat nach einem der Patentansprüche 1-4, dadurch gekennzeichnet, dass dieses 34 Gew.-% Bismut(III)-subcitrat, 41 Gew.-% Amoxycillin-Trihydrat, 1 Gew.-% Polyvinylpyrrolidon, 14 Gew.-% mikrokristalline Cellulose, 7 Gew.-% Natriumcarboxymethylcellulose, und 3 Gew.-% eines Gemisches bestehend aus Magnesiumstearat und kolloidaler Kieselsäure (im Gewichtsverhältnis 3:2) besteht.

**6.** Verwendung des Arzneipräparats nach einem der Patentansprüche 1-5 zur Herstellung eines Arzneimittels zur Behandlung von Helicobacter pylori bedingten gastroduodenalen Erkrankungen, wie Gastritis, Magenulcus, Duodenalulcus oder nicht-ulzeröser Dyspepsie.

## Claims

**1.** Solid pharmaceutical preparation in tablet form, containing as active substances a combination of bismuth (III)-citrate and amoxycillin, characterised in that (i) as bismuth(III)-citrate a bismuth(III)-subcitrate and as amoxycillin the amoxycillin-trihydrate are used, whereby in said tablet the bismuth(III)-subcitrate is present in a concentration of 32-36% by weight and the amoxycillintrihydrate is present in a concentration of 39-43% by weight, the remaining portion of the tablet consisting of a binder, a filler, a blowing agent and/or a flowing agent resp. lubricant.

**2.** Pharmaceutical preparation according to claim 1, characterised in that said preparation contains as a filler microcrystalline cellulose or lactose or a mixture thereof, as a binder polyvinylpyrrolidone, cellulose derivatives, preferably hydroxypropylmethylcellulose, gelatine or polyvinylpyrrolidone-vinylacetate-copolymers or a mixture thereof, as a blowing agent sodium carboxymethylcellulose, starch or starch derivatives, cross-linked methacrylic acid divinyl-benzene or cross-linked polyvinylpyrrolidone or a mixture thereof and as a lubricant resp. flowing agent colloidal silicic acid, metallic soaps, such as magnesium stearate, hydrated triglycerides or talcum or a mixture thereof.

**3.** Pharmaceutical preparation according to claim 1 or 2, characterised in that said preparation contains as auxiliary compounds a mixture of polyvinylpyrrolidone, microcrystalline cellulose, sodium carboxymethylcellulose, magnesium stearate and colloidal silicic acid.

**4.** Pharmaceutical preparation according to any one of the claims 1-3, characterised in that said preparation contains 0,1-10% by weight of the binder, 5-25% by weight of the filler, 1-15% by weight of the blowing agent, 0,1-6% by weight of the flowing agent resp. lubricant.

**5.** Pharmaceutical preparation according to any one of the claims 1-4, characterised in that said preparation comprises 34% by weight bismuth(III)-subcitrate, 41% by weight amoxycillin-trihydrate, 1% by weight polyvinylpyrrolidone, 14% by weight microcrystalline cellulose, 7% by weight sodium carboxymethylcellulose and 3% by weight of a mixture of magnesium stearate and colloidal silicic acid (in a weight ratio of 3:2).

**6.** The use of the pharmaceutical preparation according to any one of the claims 1-5, for the production of a medicament for the treatment of gastro duodenal illnesses caused by helicobacter pylori, such as gastritis, stomach ulcer, duodenal ulcer or non-ulcerous dyspepsia.

## Revendications

**1.** Une préparation pharmaceutique solide sous forme de comprimé, comprenant comme substances actives une combinaison de bismuth(III)- citrate et amoxycilline, caractérisée en ce que (i) on utilise en tant que bismuth (III)-citrate un bismuth(III)-subcitrate et en tant qu'amoxycilline de l'amoxycilline-trihydrate, où le bismuth(III)-subcitrate est présent dans le comprimé en une concentration comprise entre 32 et 36 % en poids et l'amoxycilline-trihydrate en une concentration comprise entre 39 et 43 % en poids, et la part restante du comprimé consiste en un agglutinant, une charge, un agent porophore et/ ou un agent d'écoulement resp. un lubrifiant.

**2.** Une préparation pharmaceutique selon la revendication 1, caractérisée en ce que la dite préparation contient en tant que charge de la cellulose microcristalline ou lactose ou leur mélange, en tant qu'agglutinant du poly(vinylpyrrolidone), des dérivés de cellulose, de préférence hydroxypropylméthylcellulose, gélatine ou des poly(vinylpyrrolidone)-vinylacétatecopolymères ou leur mélange, en tant qu'agent porophore du carboxyméthylcellulose de sodium, amidon ou dérivés d'amidon, acide méthylacrylique-divinylbenzène réticulé ou poly(vinylpyrrolidone) réticulé ou leur mélange et en tant qu'agent d'écoulement resp. lubrifiant de l'acide silicique colloïdale, des savons métalliques, tels que stéarate de magnésium, triglycérides hydratés ou talc ou leur mélange.

**3.** Une préparation pharmaceutique selon l'une quelconque des revendications 1 ou 2, caractérisée en ce que la dite préparation comprend en tant qu'adjuvants un mélange de poly(vinylpyrrolidone), cellulose microcristalline, carboxyméthylcellulose de sodium, stéarate de magnésium et acide silicique colloïdale.

**4.** Une préparation pharmaceutique selon l'une quelconque des revendications 1 à 3, caractérisée en ce que la dite préparation contient 0.1-10 % en poids d'agglutinant, 5-25 % en poids de charge, 1-15 % en poids d'agent porophore, 0.1-6 % en poids d'agent d'écoulement resp. lubrifiant.

5. Une préparation pharmaceutique selon l'une quelconque des revendications 1 à 4, caractérisée en ce que la dite préparation comprend 34 % en poids de bismuth(III)-subcitrate, 41 % en poids d'amoxycilline-trihydrate, 1 % en poids de poly(vinylpyrrolidone), 14 % en poids de cellulose microcristalline, 7 % en poids de carboxyméthylcellulose de sodium et 3 % en poids d'un mélange de stéarate de magnésium et d'acide silicique colloïdale (dans une proportion en poids de 3:2).

6. L'utilisation de la préparation pharmaceutique selon l'une quelconque des revendications 1 à 5 pour la fabrication d'un médicament pour le traitement de maladies gastro-duodénales dues au helicobacter pylori, telles que gastrites, ulcère d'estomac, ulcère duodénal ou dyspepsie non-ulcéreuse.

Abbildung 1: Plasmakonzentration von Wismut

Wismut ($\mu$g/l)

+ Prob. 1   * Prob. 2   ■ Prob. 3   ◆ Prob. 4

▲ Prob. 5   ● Prob. 6   ▼ Prob. 7   ✳ Prob. 8

Wismut ($\mu$g/l)

▨ Mittelwert n=8   ☐ SD

Sammelperiode

0: Vor        1: 0h bis 2h
2: 2h bis 4h   3: 4h bis 6h

Abbildung 2: Wismutkonzentration im Harn

Wismut (µg)

+ Prob. 1  ✳ Prob. 2  ▪ Prob. 3  ◆ Prob. 4
▲ Prob. 5  ● Prob. 6  ▼ Prob. 7  ✫ Prob. 8

Wismut (µg/l)

Mittelwert n=8  ☐ SD

Sammelperiode

0: Vor   1: 0h bis 2h   2: 2h bis 4h   3: 4h bis 6h   4: Gesamtausscheidung 0 bis 6h

Abbildung 3: Gesamtwismut im Harn

Abbildung 4: Plasmakonzentration von Amoxycillin

Abbildung 5

EP 0 624 373 B1

| | Therapiegruppe | | | |
|---|---|---|---|---|
| | Bismutcitrat + Amoxycillin | | Bismutcitrat | |
| | Anzahl | % | Anzahl | % |
| Antrum: HP-Befall Tag 0 | | | | |
| keine | 1 | 2.9 | 0 | 0 |
| leicht | 0 | 0 | 3 | 8.8 |
| mässig | 23 | 67.6 | 23 | 67.6 |
| stark | 10 | 29.4 | 8 | 23.5 |
| Antrum: HP-Befall Tag 14 | | | | |
| keine | 31 | 88.6 | 19 | 54.3 |
| leicht | 3 | 8.6 | 11 | 31.4 |
| mässig | 1 | 2.9 | 5 | 14.3 |
| Antrum: HP-Befall Tag 42 | | | | |
| keine | 22 | 62.9 | 8 | 24.2 |
| leicht | 9 | 25.7 | 11 | 33.3 |
| mässig | 3 | 8.6 | 14 | 42.4 |
| stark | 1 | 2.9 | 0 | 0 |

**Tab. 1:** Histologischer Nachweis von Helicobacter pylori im Antrum zu Beginn der Studie, nach 2 und nach 6 Wochen

| | Therapiegruppe | | | |
|---|---|---|---|---|
| | Bismutcitrat + Amoxycillin | | Bismutcitrat | |
| | Anzahl | % | Anzahl | % |
| **Corpus: HP-Befall Tag 0** | | | | |
| leicht | 18 | 56.3 | 19 | 61.3 |
| mässig | 13 | 40.6 | 11 | 35.5 |
| stark | 1 | 3.1 | 1 | 3.2 |
| **Corpus: HP-Befall Tag 14** | | | | |
| keine | 29 | 93.5 | 23 | 74.2 |
| leicht | 2 | 6.5 | 6 | 19.4 |
| mässig | 0 | 0 | 2 | 6.5 |
| **Corpus: HP-Befall Tag 42** | | | | |
| keine | 22 | 68.8 | 11 | 37.9 |
| leicht | 7 | 21.9 | 12 | 41.4 |
| mässig | 3 | 9.4 | 6 | 20.7 |

Tab. 2: Histologischer Nachweis von Helicobacter pylori im Corpus zu Beginn der Studie, nach 2 und nach 6 Wochen

EP 0 624 373 B1

| Lokalisation | Zeitpunkt | Bismutcitrat+ Amoxycillin | Bismutcitrat | Signifikanz | |
|---|---|---|---|---|---|
| Antrum | Tag 0 | 2.24 ± 0.61 | 2.15 ± 0.56 | P = 0.4 | NS |
| Corpus | Tag 0 | 1.47 ± 0.57 | 1.42 ± 0.56 | P = 0.7 | NS |
| Antrum | Tag 14 | 0.14 ± 0.43 | 0.60 ± 0.74 | P = 0.002 | S |
| Corpus | Tag 14 | 0.06 ± 0.25 | 0.32 ± 0.60 | P = 0.04 | S |
| Antrum | Tag 42 | 0.51 ± 0.78 | 1.18 ± 0.81 | P = 0.0007 | S |
| Corpus | Tag 42 | 0.41 ± 0.67 | 0.83 ± 0.76 | P = 0.02 | S |

Tab. 3:  Histologischer Verlauf des Schweregrades des HP-Befalls in Antrum und Corpus (NS=nicht signifikant, S=signifikant)

EP 0 624 373 B1

| | Therapiegruppe | | | |
|---|---|---|---|---|
| | Bismutcitrat+Amoxycillin | | Bismutcitrat | |
| . | Anzahl | % | Anzahl | % |
| Ureasetest 1 | | | | |
| positiv | 35 | 100 | 35 | 100 |
| Ureasetest 2 | | | | |
| negativ | 30 | 85.7 | 19 | 54.3 |
| positiv | 5 | 14.3 | 16 | 45.7 |
| Ureasetest 3 | | | | |
| negativ | 22 | 62.9 | 8 | 22.9 |
| positiv | 13 | 37.1 | 27 | 77.1 |

**Tab. 4:** Nachweis von Helicobacter pylori mittels **Urease** Schnelltest

EP 0 624 373 B1

| | Therapiegruppe | | | |
|---|---|---|---|---|
| | Bismutcitrat+Amoxycillin | | Bismutcitrat | |
| . | Anzahl | % | Anzahl | % |
| Wirksamkeit Arzt | | | | |
| schlecht | 4 | 11.4 | 2 | 5.7 |
| mässig | 1 | 2.9 | 8 | 22.9 |
| gut | 5 | 14.3 | 6 | 17.1 |
| sehr gut | 25 | 71.4 | 19 | 54.3 |
| Wirksamkeit Patient | | | | |
| schlecht | 1 | 2.9 | 0 | 0 |
| mässig | 4 | 11.4 | 8 | 22.9 |
| gut | 0 | 0 | 4 | 11.4 |
| sehr gut | 30 | 85.7 | 23 | 65.7 |

Tab. 5:  Beurteilung der Wirksamkeit durch Arzt und Patienten am
Ende der Studie

EP 0 624 373 B1

|  | Therapiegruppe | | | |
|  | Bismutcitrat + Amoxycillin | | Bismutcitrat | |
|  | Anzahl | % | Anzahl | % |
|---|---|---|---|---|
| **Wirksamkeit Arzt** |  |  |  |  |
| gut | 5 | 14.3 | 0 | 0 |
| sehr gut | 30 | 85.7 | 35 | 100 |
| **Wirksamkeit Patient** |  |  |  |  |
| gut | 3 | 8.6 | 0 | 0 |
| sehr gut | 32 | 91.4 | 35 | 100 |

**Tab. 6:** Beurteilung der Verträglichkeit durch Arzt und Patienten am Ende der Studie

EP 0 624 373 B1